(19) Europäisches Patentamt
European Patent Office
Office européen des brevets

(11) Publication number: **0 664 295 A1**

(12) **EUROPEAN PATENT APPLICATION**

(21) Application number: **94810039.1**

(22) Date of filing: **24.01.94**

(51) Int. Cl.[6]: **C07D 493/06**, C07C 65/40, C07C 69/738, C07D 307/32, A61K 31/19, A61K 31/335, A61K 31/215, C12P 15/00, C12P 17/04, C12P 17/18, //(C07D493/06,313:00,307:00), (C12P17/18,C12R1:79)

The microorganism(s) has (have) been deposited with Deutsche Sammlung für Mikroorganismen und Zellkulturen under number DSM 7243.

Claim 11 is deemed to be abandoned due to non-payment of the claims fee (Rule 31 (2) EPC).

(83) Declaration under Rule 28(4) EPC (expert solution)

(43) Date of publication of application: **26.07.95 Bulletin 95/30**

(84) Designated Contracting States: **GB**

(71) Applicant: **CIBA-GEIGY AG**
**Klybeckstrasse 141**
**CH-4002 Basel (CH)**

(72) Inventor: **Fredenhagen, Andreas Dr.**
**Hackbergstrasse 42**
**CH-4125 Riehen (CH)**
Inventor: **Petersen, Frank Dr.**
**Im Altweg 29**
**D-79541 Lörrach (DE)**

(54) **Anthraquinone derivatives, process for their preparation and their use as medicaments.**

(57) Compounds of the formula

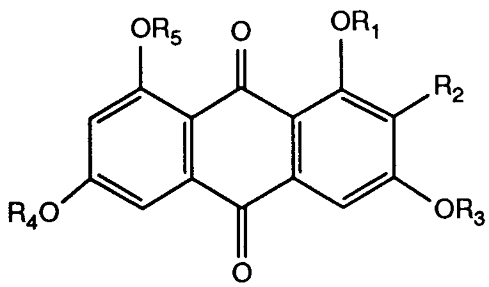

(I),

wherein $R_1$ is hydrogen or alkyl;
$R_2$ is 2-oxo-tetrahydrofur-3-yl, $CH(R_6)-(CH_2)_2-X-CH_3$ wherein $R_6$ is carboxy or alkoxycarbonyl and X is C(=O) or $CH(OR_7)$ wherein $R_7$ is hydrogen or alkyl, or $R_2$ is $CH(R_6)-CH_2-CH_2-OR_7$ wherein $R_6$ and $R_7$ have the meanings given above;
and $R_3$, $R_4$ and $R_5$ are each independently of the other hydrogen or alkyl;
or wherein $R_1$, $R_4$ and $R_5$ have the meanings given above and $R_2$ and $R_3$ together form 2,3 epoxy-3-carboxy-pentan-2,5-diyl or 2,3 epoxy-3-alkoxycarbonyl-pentan-2,5-diyl, the $C_2$-atom of said pentan-2,5-diyl binding to the oxygen;
or wherein the partial structure

EP 0 664 295 A1

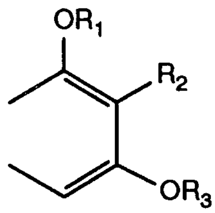

represents the group of the formula

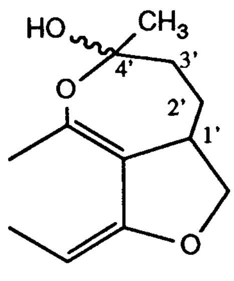

(II)

are protein kinase inhibitors and useful in the therapeutic treatment of the animal and human body.

The present invention relates to a compound of the formula

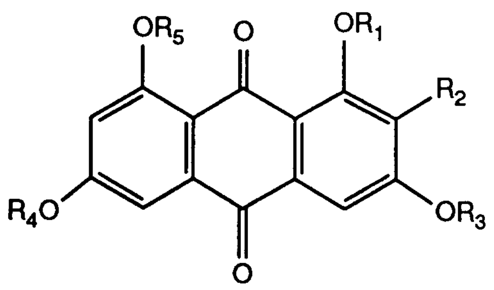

(I),

wherein $R_1$ is hydrogen or alkyl;
$R_2$ is 2-oxo-tetrahydrofur-3-yl, $CH(R_6)$-$(CH_2)_2$-X-$CH_3$ wherein $R_6$ is carboxy or alkoxycarbonyl and X is C-(=O) or $CH(OR_7)$ wherein $R_7$ is hydrogen or alkyl, or $R_2$ is $CH(R_6)$-$CH_2$-$CH_2$-$OR_7$ wherein $R_6$ and $R_7$ have the meanings given above;
and $R_3$, $R_4$ and $R_5$ are each independently of the other hydrogen or alkyl;
or wherein $R_1$, $R_4$ and $R_5$ have the meanings given above and $R_2$ and $R_3$ together form 2,3-epoxy-3-carboxy-pentan-2,5-diyl or 2,3 epoxy-3-alkoxycarbonyl-pentan-2,5-diyl, the $C_2$-atom of said pentan-2,5-diyl binding to the oxygen;
or wherein the partial structure

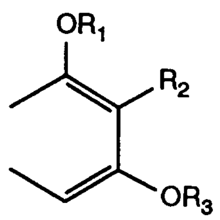

represents the group of the formula

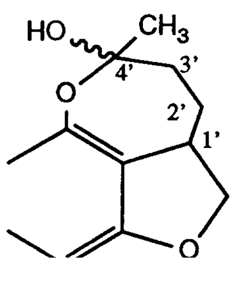

(II);

to salts thereof when salt-forming groups are present, to processes for the preparation of those compounds, to pharmaceutical compositions comprising those compounds, and to the use of those compounds for the therapeutic treatment of the human or animal body or for the preparation of pharmaceutical compositions.

The general definitions used hereinbefore and hereinafter have primarily the following meanings, unless defined differently:

Alkyl denotes, in particular, $C_1$-$C_7$alkyl and is, for example, methyl, ethyl, n-propyl, isopropyl, n-butyl, isobutyl, sec-butyl, tert-butyl and furthermore embraces appropriate pentyl, hexyl and heptyl radicals. $C_1$-$C_4$Alkyl is preferred. Particularly preferred is methyl.

Alkoxycarbonyl is, in particular, $C_1$-$C_7$alkoxycarbonyl and is, for example, methoxy-, ethoxy-, propyloxy-, isopropyloxy-, n-butyloxy-, isobutyloxy- and tert. butyloxy-or pivaloyloxy-carbonyl. $C_1$-$C_4$Alkoxycarbonyl is preferred. Particularly preferred is methoxycarbonyl.

The expression "lower" means that corresponding groups and compounds in each case contain in particular not more than 7, preferably not more than 4, carbon atoms.

Salts of compounds according to the invention having salt-forming groups are especially pharmaceutically acceptable, non-toxic salts. Compounds of formula I having a carboxy group, form, for example, metal or ammonium salts, such as alkali metal and alkaline earth metal salts, for example sodium, potassium, magnesium or calcium salts, and also ammonium salts with ammonia or suitable organic amines, such as lower alkylamines, for example triethylamine, hydroxy-lower alkylamines, for example 2-hydroxyethylamine, bis(2-hydroxyethyl)amine or tris(2-hydroxyethyl)amine, basic aliphatic esters of carboxylic acids, for example 4-aminobenzoic acid 2-diethylaminoethyl ester, lower alkyleneamines, for example 1-ethylpiperidine, cycloalkylamines, for example dicyclohexylamine, or benzylamines, for example N,N'-dibenzylethylenediamine, dibenzylamine or benzyl-$\beta$-phenethylamine. Furthermore, the compounds according to the invention which have an acidic phenolic hydroxyl group ($R_1$, $R_3$, $R_4$ and/or $R_5$ = hydrogen) form salts with bases, for example, alkali metals, such as sodium or potassium salts.

For the purposes of isolation or purification it is also possible to use pharmaceutically unacceptable salts. Only the pharmaceutically acceptable, non-toxic salts are used therapeutically and these are therefore preferred.

The compounds of the formula I that contain an asymmetric carbon atom can each be in the form of a racemate or in the form of an R- or S-enantiomer. The invention relates to all these forms and, for example, also to diastereomers and mixtures thereof which may occur when there are two or more asymmetric centres in the molecule.

The compounds according to the invention have valuable, especially pharmacologically acceptable, properties. In particular they exhibit specific inhibitory activities that are of pharmacological interest. They act especially as tyrosine protein kinase inhibitors and exhibit, for example, a potent inhibition of the tyrosine kinase activity of the receptor for the epidermal growth factor (EGF) and the c-erbB2 kinase. These receptor-specific enzyme activities play a key role in the signal transmission in a large number of mammal cells, including human cells, especially epithelial cells, cells of the immune system and cells of the central ad peripheral nervous system. The EGF-induced activation of the receptor-associated tyrosine protein kinase (EGF-R-PTK) is in various cell types a prerequisite for cell division and thus for the proliferation of a cell population. The addition of EGF-receptor-specific tyrosine kinase inhibitors therefore inhibits the reproduction of those cells.

The inhibition of EGF-receptor-specific tyrosine protein kinase (EGF-R-PTK) can be demonstrated, for example, using the method of C. House et al., Europ. J. Biochem. 140, 363-367(1984). Furthermore, for example, the compounds of the invention also exhibit an inhibition of the cell growth of an EGF-dependent cell line, for example the epidermoid mouse keratinocyte cell line. In order to measure this inhibition of cell growth, the EGF-stimulated cell proliferation of epidermal Balb/MK keratinocytes is used (description of the method in Meyer, T., et al., Int. J. Cancer 43, 851-856 (1989)). Those cells are to a high degree dependent upon the presence of EGF for proliferation (Weissmann, B. E., Aaronson, S. A., Cell 32, 599 (1983)). In order to carry out the test, Balb/MK cells (10 000/well) are transferred to 96-well microtitre plates and incubated overnight. The test substances (dissolved in DMSO) are added in various concentrations (in dilution series) so that the final concentration of DMSO is no greater than 1 %. After the addition, the plates are incubated for three days, during which time the control cultures without the test substance are able to undergo at least three cell division cycles. The growth of the MK cells is measured by means of methylene blue staining.

In addition to inhibiting EGF-R-PTK, the compounds according to the invention also inhibit other tyrosine kinases that are involved in signal transmission mediated by trophic factors, for example the v-abl-kinase, kinases from the family of c-src-kinases and the c-erbB2 kinase (HER-2), and other kinases with a different specificity, for example serine/threonine kinases.

The inhibition of the c-erbB2 tyrosine kinase (HER-2) can be demonstrated, for example, analogously to the method of C. House et al., Europ. J. Biochem. 140, 363-367 (1984) used for EGF-R-PTK. The c-erbB2 kinase can be isolated, and its activity determined, in accordance with known protocols, for example in accordance with T. Akiyama et al., Science 232, 1644 (1986).

The inhibition of the v-abl tyrosine kinase can be demonstrated, for example, analogously to the assay described by N. Lydon et al. Oncogene Res. 5, 161-73 (1990).

The inhibition of the c-src tyrosine kinase can be measured according to the method disclosed by J.F.Geissler et al., J. Biol. Chem 265, 22255-61 (1990).

The compounds according to the invention are particularly suitable for the inhibition of processes mediated by these and related tyrosine kinases.

The compounds according to the invention are therefore useful, for example, in the treatment of benign or malignant tumours. They are able to bring about the regression of tumours and to prevent the formation of tumour metastases and the growth of micro-metastases. In particular, they can be used in epidermal

hyperproliferation (psoriasis), in the treatment of neoplasias of epithelial character, for example mammary carcinoma, and in leukaemias, particularly chronic myelocytic and acute lymphoblastic leukaemia. In addition, the compounds can be used in the treatment of diseases of the immune system and in the treatment of inflammation insofar as protein kinases are involved in those disorders. The compounds can also be used in the treatment of disorders of the central or peripheral nervous system insofar as signal transmission by protein kinases is involved.

The compounds according to the invention can be used both on their own and in combination with other pharmacologically active substances, for example together with (a) inhibitors of enzymes of polyamine synthesis, (b) inhibitors of protein kinase C, (c) inhibitors of other tyrosine kinases, (d) cytokines, (e) negative growth regulators, for example TGF-$\beta$ or IFN-$\beta$, (f) aromatase inhibitors, (g) anti-oestrogens or (h) cytostatics.

Preferably, the invention relates to a compound of the formula I, wherein $R_1$ is hydrogen or alkyl; $R_2$ is 2-oxo-tetrahydrofur-3-yl, $CH(R_6)-(CH_2)_2-X-CH_3$ wherein $R_6$ is carboxy or lower alkoxycarbonyl and X is C-(=O) or $CH(OR_7)$ wherein $R_7$ is hydrogen or alkyl or $R_2$ is $CH(R_6)-CH_2-CH_2-OR_7$ wherein $R_6$ and $R_7$ have the meanings given above;
and $R_3$, $R_4$ and $R_5$ are each independently of the other hydrogen or alkyl; or wherein $R_1$, $R_4$ and $R_5$ have the meanings given above and $R_2$ and $R_3$ together form 2,3-epoxy-3-carboxy-pentan-2,5-diyl or 2,3 epoxy-3-alkoxycarbonyl-pentan-2,5-diyl,;
with the provisio that at least either of $R_1$ or $R_5$ is hydrogen.

More preferably, the invention relates to a compound of the formula I, wherein $R_1$ is hydrogen or methyl; $R_2$ is 2-oxo-tetrahydrofur-3-yl, $CH(R_6)-(CH_2)_2-X-CH_3$ wherein $R_6$ is carboxy or methoxycarbonyl and X is C(=O) or $CH(OR_7)$ wherein $R_7$ is hydrogen or methyl, or $R_2$ is $CH(R_6)-CH_2-CH_2-OR_7$ wherein $R_6$ and $R_7$ have the meanings given above; and $R_3$, $R_4$ and $R_5$ are each independently of the other hydrogen or methyl; or
wherein $R_1$, $R_4$ and $R_5$ have the meanings given above and $R_2$ and $R_3$ together form 2,3-epoxy-3-carboxy-pentan-2,5-diyl or 2,3 epoxy-3-alkoxycarbonyl-pentan-2,5-diyl,;
with the provisio that at least either of $R_1$ or $R_5$ is hydrogen.

Most preferably, the invention relates to a compound of the formula I, wherein $R_1$ is hydrogen ; $R_2$ is 2-oxo-tetrahydrofur-3-yl, $CH(R_6)-(CH_2)_2-X-CH_3$ wherein $R_6$ is carboxy and X is C(=O), or $CH(R_6)-CH_2-CH_2-OR_7$ wherein $R_6$ and $R_7$ have the meanings given above; and $R_3$, $R_4$ and $R_5$ are each hydrogen.

The invention particularly relates to the compounds decribed in the Examples.

The invention further relates to a process for the production of a compound of the invention, said process comprising culturing the novel strain Paecilomyces carneus P-177, or a culture derivable therefrom, in a suitable nutrient medium, isolating said compound from the culture broth and, optionally, converting said compound into another compound of the invention or into a salt.

The novel strain Paecilomyces carneus P-177 Brown & Smith was isolated from a soil sample taken from jungle region in Bolivia and deposited in accordance with the provisions of the Budapest Treaty with the Deutsche Sammlung für Mikroorganismen (DSM) in Braunschweig under accession no. DSM 7243 on September 16, 1992. The features of said strain are given in Example 1.

A derivable culture in the above sense will be understood as meaning any culture which in addition has the features of the deposited culture essential for carrying out the process of the invention, i.e. mainly a derived culture, especially a culture whose microorganisms contain the same structural genes as those of DSM 7243 essential for the formation of a compound of the formula I. A derivable culture will also be understood as meaning any culture whose microorganisms contain an equivalent of the structural genes of strain DSM 7243, which equivalent is possible owing to the degeneration of the genetic code. In particular, a derivable culture will be understood as meaning any culture which contains microorganisms of the species Paecilomyces carneus, which are capable of producing a compound of the formula I. The term "derivable culture of the strain DSM 7243" also includes all mutants of said strain which are capable of producing a compound of formula I.

As nutrient medium it is preferred to use a solid medium or more preferably an aqueous solution or suspension comprising at least one suitable source of carbon and at least one suitable source of nitrogen and preferably also mineral salts. Suitable carbon sources are glycerol, assimilable carbohydrates such as cyclitols like mannite, monosaccharides, especially glucose, disaccharides, particularly lactose, polysaccharides, e.g. starch, and also corresponding carbohydrate-containing industrial raw materials such as sugar beet or sugar cane molasses. Suitable nitrogen sources are for example amino acids, especially the natural $\alpha$-amino acids, peptides, proteins and the degradation products thereof such as peptones and tryptones, but also ammonia salts and nitrates. Furthermore, industrial nitrogen containing raw materials such as meat extract, yeast extract, yeast autolysate or casein hydrolysate are usable. Also suitable are mixed carbon and

nitrogen sources such various plant seeds that are used in the form of aqueous extracts, meal or mash, for example of beans, such as soybeans, cereal grains, wheat, maize ("corn steep liquor") and especially peanut meal; further cotton seed meal and malt extract. Additionally to ammonia salts like ammonia chloride, sulfate, or nitrate, and other nitrates the medium may comprise mineral salts. Suitable mineral salts include chlorides, carbonates, sulfates and phosphates of alkali metals and, more particularly, alkaline alkaline earth metals, for example, sodium, potassium, and preferably, magnesium and calcium, as well as salts comprising trace elements like iron, zinc, and manganese. It is also advantageous to add further growth-promoting substances, especially vitamins, conveniently in the form of commercially available standard vitamin solutions. It is preferred to use the nutrient media employed in the Examples herein.

The production medium is sterilized and inoculated with the production strain according to common microbiological methods.

The cultivation is carried out aerobically, i.e. conveniently in resting surface cultures, or preferably submerged with shaking or stirring while inducing air or oxygen into a shake flask or fermenter. Fermentation is performed in the neutral pH range, i.e. at about pH 6-8, preferably at about pH 8. The temperature ranges from about 20 to about 35 °C, preferably from about 27 to about 33 °C, especially preferred is a temperature of about 33 °C. The fermentation period is from about 2 to about 7 days, preferably from about 4 to about 6 days. Preferably, the cultivation is carried out in two steps, first a liquid preculture is prepared and after a suitable incubation period of 1 and 5 days, preferably 2 days, 1-10 % related to the volume of the production medium is used as an inoculum of the production medium.

The compounds of the invention are isolated from the culture broth in a manner known per se. For example, after separating the biomass from the culture broth, e.g. by centrifugation or by filtration, isolation of a compound of the invention may comprise extraction with a suitable organic, dipolar aprotic solvent, one or more chromatographic steps and/or crystallisation. Suitable dipolar aprotic solvents include esters of aliphatic acids, typically alkyl esters, such as methyl or ethyl esters, of acetic acid. Chromatography may involve adsorption and partition chromatography including reversed phase chromatography, gel filtration and for acidic compounds ion exchange chromatography. Stationary phases useful therefor are e.g. silica gel, XAD-1180, XAD-16, diaion HP-20, charcoal, or chemically modified silica gel, such as C-8 or C-18-modified silica gel, gel filtration resins, for example modified dextrans such as Sephadex, e.g. Sephadex G-10 or Sephadex LH-20, or Fractogel HW-40, and ion-exchange resins including Fractogel DEAE-650 and DEAE-Sephadex. Preferably, crystallization is effected from a lower alkanol such as ethanol or particularly methanol.

Preferably, isolation of a compound of the invention from the culture broth of Paecilomyces carneus comprises

- adjusting the culture supernatant to an acidic pH, preferably a pH of about 5, with a suitable acid, e.g. a mineral acid such as HCl.
- column chromatography on silica gel, and optionally
- crystallization.

If appropriate, the compounds of the invention may also be obtained by chemical de novo synthesis according to methods known in the art.

If desired, a compound of the formula I obtainable from the culture broth of Paecilomyces carneus can be converted in a manner known per se into a different compound of the formula I, converted into a salt and/or a resulting mixture of isomers can be separated.

For example, compounds of the formula I wherein $R_1$, $R_3$, $R_4$, $R_5$ and/or $R_7$ are hydrogen can be converted into a compound wherein $R_1$, $R_3$, $R_4$, $R_5$ and/or $R_7$ are alkyl, i.e. the hydroxy group is etherified. Suitable etherifying agents are, for example, appropriate diazo compounds, such as diazo-lower alkanes, for example diazomethane, diazoethane or diazo-n-butane. These reagents are employed in the presence of a suitable inert solvent, such as an aliphatic, cycloaliphatic or aromatic hydrocarbon, such as hexane, cyclohexane, benzene or toluene, a halogenated aliphatic hydrocarbon, for example methylene chloride, or an ether, such as a di-alkyl ether, for example diethyl ether, or a cyclic ether, for example tetrahydrofuran or dioxan, or a solvent mixture, and depending on the diazo reagent, while cooling, at room temperature or while slightly heating, and also, if necessary, in a closed vessel and/or under an inert gas atmosphere, for example a nitrogen atmosphere.

Further suitable etherifying agents are esters of corresponding alcohols, especially those with strong inorganic or organic acids, such as mineral acids, for example hydrohalic acids, such as hydrochloric acid, hydrobromic acid or hydroiodic acid, and also sulphuric acid or halosulphuric acid. Such esters are, inter alia, alkyl halides, di-alkyl sulphates, such as dimethyl sulphate, also fluorosulphonic acid esters, such as alkyl esters, for example fluorosulphonic acid methyl ester or optionallly halo-substituted methane-sulphonic alkyl esters, for example trifluoromethanesulphonic acid methyl ester. They are usually used in the

presence of an inert solvent, such as an optionally halogenated, such as chlorinated, aliphatic, cycloaliphatic or aromatic hydrocarbon, for example methylene chloride, an ether, such as dioxan or tetrahydrofuran, or a mixture. At the same time, suitable condensation agents are preferably used, such as alkali metal carbonates or bicarbonates (usually together with a sulphate), or organic bases, such as, usually sterically hindered, tri-alkylamines, for example N,N-diisopropyl-N-ethylamine, the process being carried out while cooling, at room temperature or while heating, for example at temperatures from approximately - 20 °C to approximately -50 °C and, if necessary, in a closed vessel and/or in an inert gas atmosphere, for example a nitrogen atmosphere.

The above-described etherification reactions can be considerably accelerated by phase transfer catalysis (Dehmlow, Angewandte Chemie 5, 187 (1974)).

Further etherifying agents are suitable acetal compounds, e.g. gem-di-lower alkoxy-lower alkanes, such as 2,2-dimethoxy propane, which are used in the presence of strong organic sulphonic acids and a suitable solvent, such as di-lower alkyl sulphoxide or lower alkylene sulphoxide.

Further etherifying agents are corresponding tri-substituted oxonium salts (so-called Meerwein salts), disubstituted carbenium or halonium salts, in which the substituents are the etherifying radicals, for example tri-lower alkyloxonium salts, and also di-lower alkoxycarbenium salts or di-lower alkylhalonium salts, especially the corresponding salts with complex, fluorine -containing acids, such as the corresponding tetrafluoroborates, hexafluorophosphates, hexafluoroantimonates or hexachloroantimonates. Such reagents are, for example, trimethyloxonium or triethyloxonium, hexafluoroantimonate, hexachloroantimonate, hexafluorophosphate or tetrafluoroborate, dimethoxycarbenium hexafluorophosphate or dimethylbromonium hexafluoroantimonate. These etherifying agents are used preferably in an inert solvent, such as an ether or a halogenated hydrocarbon, for example diethyl ether, tetrahydrofuran or methylene chloride, or in a mixture thereof, if necessary in the presence of a base, such as an organic base, for example a preferably sterically hindered tri-lower alkylamine, for example N,N-diisopropyl-N-ethyl-amine, and while cooling, at room temperature and, preferably, at elevated temperature, for example at from approximately 20 °C to approximately 100 °C, if necessary in a closed vessel and/or in an inert gas atmosphere, for example a nitrogen atmosphere.

Furthermore, etherifying agents suitable for introducing a methyl radical are precursors of dimethylsulfoxonium methylide such as trimethylsulfoxonium chlorid and trimethylsulfoxoniumjodid (E.J. Corey et al., J. Am. Chem. Soc. 87, 1353 (1965).

Inversely, corresponding ethers, such as alkoxy compounds can be cleaved, for example, by means of strong acids, such as mineral acids, for example the hydrohalic acids.

Furthermore, a carboxy group in a compound of the formula I can be converted into a alkoxycarbonyl group (esterified carboxyl group), for example, by treating with an alcohol, such as a lower alkanol, in the presence of a suitable esterifying agent, such as an acid reagent, for example an inorganic or organic acid or a Lewis acid, for example zinc chloride, or a condensing agent which combines with water, for example a carbodiimide, such as N,N'-dicyclohexylcarbodiimide, or by treating with a diazo reagent, such as with a diazo-lower alkane mentioned above. This can also be obtained if compounds of the formula I in which the carboxy group is present in free form or in salt form, such as ammonium salt or metal salt, for example alkali metal salt, such as sodium salt or potassium salt form are treated with a reactive ester of a lower alkanol, e.g. esters mentioned above. Esterification of the carboxy group can also be achieved by treatment with a gem-di-lower alkoxy-lower alkane, e.g. 2,2-dimethoxy propane.

Compounds of the invention which contain an esterified carboxyl group as a substituent can be transesterified into another ester compound of the invention , for example by treating with an alcohol, customarily a higher appropriate alcohol than that of the esterified carboxyl group in the starting material, in the presence of a suitable transesterifying agent, such as a basic agent, for example and alkali metal ($C_1$-$C_7$)alkanoate, ($C_1$-$C_7$)alkanolate or cyanide, or a suitable acid agent, if appropriate with removal of the resulting alcohol, for example by distillation.

A compound of the formula I wherein $R_1$, $R_3$, $R_4$ and $R_5$ have the meanings given above and $R_2$ is $CH(R_6)$-$(CH_2)_2$-X-$CH_3$ wherein $R_6$ is carboxy or alkoxycarbonyl and X is C(=O) can be reduced to a compound wherein X is $CH(OR_7)$ wherein $R_7$ is hydrogen. The reduction of the carbonyl group is effected, for example, by means of suitable hydride reducing agents. These are especially complex metal hydrides, such as corresponding borohydrides, for example diborane, or alkali metal borohydrides, for example sodium borohydride or lithium borohydride, and also zinc borohydride, as well as organic alkali metal aluminium hydrides, such as lithium aluminium hydride, which are usually employed in the presence of solvents, especially relatively polar solvents, such as alcohols, for example lower alkanols, such as methanol or ethanol, or ethers, such as tetrahydrofuran or dioxan or solvent mixtures, and also in the presence of water-containing solvents, the reaction being carried out at temperatures of from approximately -20 °C to

approximately 80 °C, if necessary in a closed vessel and/or in an inert gas atmosphere, for example a nitrogen atmosphere.

Compounds of the invention having a carboxy group can be converted into a salt in a manner known per se, for example by treatment with suitable bases or derivatives thereof.

The novel compounds can be obtained in the form of one of the possible isomers or as mixture thereof, for example depending on the number of asymmetric carbon atoms, as pure optical isomers, such as antipodes, or as mixtures of isomers, such as racemates, diastereomeric mixtures or racemate mixtures.

Racemate mixtures which are obtained can be separately fractionated on the basis of the physicochemical difference of the components in a known manner into the pure isomers or racemates, for example by fractional crystallization. Racemates which are obtained can, furthermore, be resolved by known methods into the optical antipodes, for example by recrystallization from an optically active solvent, chromatography on chiral adsorbents, using suitable microorganisms, by cleavage with specific, immobilized enzymes, via the formation of inclusion compounds, for example using chiral crown ethers, in which case only one enantiomer is complexed, or by conversion into diastereomeric salts, for example by reaction of a basic final racemate with a optically active acid, for example tartaric or mallic acid, or sulfonic acid, for example camphorsulfonic acid, and separation of the diastereomeric mixture obtained in this manner, for example on the basis of their different solubilities, into the diastereomers, from which the desired enantiomer can be liberated by the action of suitable agents. The more active enantiomer is advantageously isolated.

The compounds may also be obtained in form of their crystals, for example include the solvent used for their crystallisation.

In view of the close relationship between a compound of formula I in free form and in the form of its salt, hereinbefore and hereinafter any reference to the free compound or its salt should be understood as including also the corresponding salt or free compound, respectively, where appropriate and expedient.

The present invention relates also to pharmaceutical compositions that comprise one of the compounds of formula I as active ingredient. Compositions for enteral, especially oral, and for parenteral administration are especially preferred. The compositions comprise the active ingredient on its own or, preferably, together with a pharmaceutically acceptable carrier. The dosage of the active ingredient depends upon the disease to be treated and upon the species, its age, weight and individual condition, and also upon the mode of administration.

Preferred is a pharmaceutical composition suitable for administration to a warm-blooded animal, especially a human, suffering from a disease responsive to the inhibition of a protein kinase, for example psoriasis or a tumour, comprising a compound of formula I, or a salt thereof when salt-forming groups are present, in an amount effective for the inhibition of the protein kinase, together with at least one pharmaceutically acceptable carrier.

The pharmaceutical compositions comprise from approximately 5 % to approximately 95 % active ingredient, dosage forms in single dose form preferably comprising from approximately 20 % to approximately 90 % active ingredient and dosage forms that are not in single dose form preferably comprising from approximately 5 % to approximately 20 % active ingredient. Unit dose forms, such as dragées, tablets or capsules, comprise from approximately 0.002g to approximately 1.0 g of active ingredient.

The pharmaceutical compositions of this invention are prepared in a manner known per se, for example by means of conventional mixing, granulating, confectioning, dissolving or lyophilising processes. For example, pharmaceutical compositions for oral use can be obtained by combining the active ingredient with one or more solid carriers, optionally granulating a resulting mixture, and, if desired, processing the mixture or granules, if appropriate with the addition of additional excipients, to form tablets or dragée cores.

Suitable carriers are especially fillers, such as sugars, for example lactose, saccharose, mannitol or sorbitol, cellulose preparations and/or calcium phosphates, for example tri-calcium phosphate or calcium hydrogen phosphate, also binders, such as starches, for example corn, wheat, rice or potato starch, methylcellulose, hydroxypropylmethylcellulose, sodium carboxymethylcellulose and/or polyvinylpyrrolidone, and/or, if desired, disintegrators, such as the above-mentioned starches, also carboxymethyl starch, cross-linked polyvinylpyrrolidone, alginic acid or a salt thereof, such as sodium alginate. Additional excipients are especially flow conditioners and lubricants, for example silicic acid, talc, stearic acid or salts thereof, such as magnesium or calcium stearate, and/or polyethylene glycol, or derivatives thereof.

Dragée cores can be provided with suitable, optionally enteric, coatings, there being used inter alia concentrated sugar solutions which may contain gum arabic, talc, polyvinylpyrrolidone, polyethylene glycol and/or titanium dioxide, or coating solutions in suitable organic solvents or solvent mixtures, or, for the production of enteric coatings, solutions of suitable cellulose preparations, such as acetylcellulose phthalate or hydroxypropylmethylcellulose phthalate. Colourings or pigments may be added to the tablets or dragée coatings, for example for identification purposes or to indicate different doses of active ingredient.

Orally administrable pharmaceutical compositions also include dry-filled capsules consisting of gelatin, and also soft, sealed capsules consisting of gelatin and a plasticiser, such as glycerol or sorbitol. The dry-filled capsules may contain the active ingredient in the form of granules, for example in admixture with fillers, such as corn starch, binders and/or glidants, such as talc or magnesium stearate, and optionally stabilisers. In soft capsules, the active ingredient is preferably dissolved or suspended in suitable liquid excipients, such as fatty oils, paraffin oil or liquid polyethylene glycols, to which stabilisers may also be added.

Other oral dosage forms are, for example, syrups prepared in customary manner which comprise the active ingredient, for example, in suspended form and in a concentration of about 5 % to 20 %, preferably about 10 %, or in a similar concentration that provides a suitable single dose, for example, when administered in measures of 5 or 10 ml. Also suitable are, for example, powdered or liquid concentrates for the preparation of shakes, for example in milk. Such concentrates may also be packaged in single dose quantities.

Suitable rectally administrable pharmaceutical compositions are, for example, suppositories that consist of a combination of the active ingredient and a suppository base. Suitable suppository bases are, for example, natural or synthetic triglycerides, paraffin hydrocarbons, polyethylene glycols or higher alkanols.

For parenteral administration there are especially suitable aqueous solutions of an active ingredient in water-soluble form, for example in the form of a water-soluble salt, or aqueous injection suspensions that contain viscosity-increasing substances, for example sodium carboxymethylcellulose, sorbitol and/or dextran, and, if desired, stabilisers. The active ingredient, optionally together with excipients, can also be in the form of a lyophilisate and can be made into a solution prior to parenteral administration by the addition of suitable solvents.

Solutions such as are used, for example, for parenteral administration can also be used as infusion solutions.

The invention relates also to a method of treating the above-mentioned pathological conditions, especially those conditions responsive to the inhibition of protein kinases. The compounds of this invention can be administered prophylactically or therapeutically, preferably in an amount effective against the said diseases, to a warm-blooded animal, for example a human, requiring such treatment, the compounds preferably being used in the form of pharmaceutical compositions. In the case of an individual having a body weight of about 70 kg the daily dose administered of a compound of this invention is from approximately 0.001 g to approximately 5 g, preferably from approximately 0.001 g to about 0.5 g for parenteral administration and approximately 0.002 g to about 1 g for oral administration.

In the following Examples which illustrate the invention but do not limit it in any form melting points are uncorrected and given in Celsius. Large scale liquid chromatography is done using a medium pressure system equipped with a Büchi pump B-681, Büchi glass columns B-685, Kontron Uvikon 725 detector (1 mm pathlength) and a Büchi B-684 fraction collector. Preparative reversed phase chromatography is performed with a Labomat VS200 gradient mixer, a Besta-HD-200 pump, a Shimadzu UV-120-02 UV detector (1 mm path) and a Büchi B-684 fraction collector. For HPLC a Spectra Physics SP8700 delivery system with Spectra Physics Focus UV/VIS detector and integrator Merck-Hitachi D-2000 is used. Columns: semipreparative silica gel: Nucleosil 100-5, 100 Å, 5 µm, 16 x 250 mm; analytical silica gel: Nucleosil 50-5, 50 Å, 5µm; 4x100mm (cartridge). The following abbreviations are used: MP = melting point; $t_R$ = retention time;

THF = tetrahydrofuran; ether = diethyl ether, MeOH = methanol.

Example 1: Production Conditions

a) Production strain: *Paecilomyces carneus* Brown & Smith P-177.

b) Origin of the production strain: isolated from a soil sample from jungle region, Bolivia.

c) Description of the production strain: Colony is colorless to pale pink, powdery and reaches 20 mm diameter in 8 days at 22 °C. On solid nutrient medium the organism grows well on LCSB agar (lactose 15 g/l, cornsteep liquor 5 g/l, peptone 5 g/l, NaCl 4 g/l, $MgSO_4$ x 7 $H_2O$ 0,5 g/l, $KH_2PO_4$ 0,6 g/l, $FeCl_3$ x 6 $H_2O$ 0,005 g/l, $CuSO_4$ x 5 $H_2O$ 0,002 g/l, agar 30 g/l, pH 4,8). The conidiophores are branched penicilloidly; phialides are cylindric and ellipsoid with narrow neck. Conidia are ellipsoid ca. 3 µm long and spiky.

d) Efficacy of the production strain:

In the supernatant of the culture broth of Paecilomyces carneus P-177 the inhibiting activity is detectable in an above-indicated enzyme test. The active substances are called paeciloquinones. The compounds of the invention are referred to as paeciloquinones A,B,D,E,F and derivatives thereof. Paeciloquinone A is

a compound of the formula I, wherein $R_1$, $R_3$, $R_4$ and $R_5$ are hydrogen and $R_2$ is 2-oxo-tetrahydrofur-3-yl; paeciloquinone B is a compound of the formula I, wherein $R_1$, $R_3$, $R_4$ and $R_5$ are hydrogen and $R_2$ is $CH(R_6)$-$(CH_2)_2$-X-$CH_3$ wherein $R_6$ is carboxy and X is C(=O); paeciloquinone D is a compound of the formula I, wherein $R_1$, $R_3$, $R_4$ and $R_5$ are hydrogen and $R_2$ $CH(R_6)$-$CH_2$-$CH_2$-$OR_7$ wherein $R_6$ is carboxy and $R_7$ is hydrogen. Paeciloquinone F is a compound of the formula I, wherein $R_1$, $R_4$ and $R_5$ are hydrogen and $R_2$ and $R_3$ together form 2,3-epoxy-3-carboxy-pentan-2,5-diyl, the $C_2$-atom of said pentan-2,5-diyl binding to the oxygen. Paeciloquinone E is a compound of the formula I, wherein $R_4$ and $R_5$ are hydrogen and the partial structure

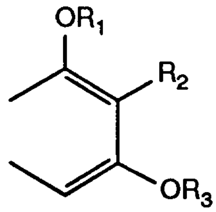

represents the group of the formula

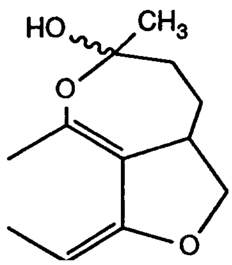 (II).

The compound designated Paeciloquinone C (2-Hydroxymethyl-1,3,6,8-tetrahydroxy-anthraquinone) is kwown in the art (S. Shibata et al. Yakugaku Zasshi 80, 620-4 (1960).

e) Accessibility of the strain: the production strain Paecilomyces carneus P-177 was deposited with the Deutsche Sammlung für Mikroorganismen (DSM) in Braunschweig under accession no. DSM 7243 on September 16, 1992.

f) Production conditions of Paeciloquinones: in shake flask the paeciloquinones are formed during growth to stationary phase and reach maximal titer after 4-5 days.

Example 2: Fermentation in flasks

For preparation of inoculation material the organism Paecilomyces carneus P-177 is incubated on LCSB agar (lactose 15 g/l, cornsteep liquor 5 g/l, peptone 5 g/l, NaCl 4 g/l, $MgSO_4$ x 7 $H_2O$ 0,5 g/l, $KH_2PO_4$ 0,6 g/l, $FeCl_3$ x 6 $H_2O$ 0,005 g/l, $CuSO_4$ x 5 $H_2O$ 0,002 g/l, agar 30 g/l, pH 4,8) for 10 days at 28°C till the sporulation is developed well. The slant culture is stored at - 20°C. One slant is used as inoculum for the preculture with following ingredients: glucose 20 g/l, Sunpro 15 g/l, $(NH_4)_2SO_4$ 3 g/l, $ZnSO_4$ x 7 $H_2O$ 0,03 g/l, $CaCO_3$ 4 g/l, pH 7,0. The preculture is incubated for 3 days at 28°C on a rotary shaker (250 rpm, amplitude 50 mm). 500 ml Erlenmeyer flasks with 1 baffle and 100 ml of the indicated medium are used.

a) 128 shake flasks with 4 baffles and 100 ml of the production medium CG-D 2.1 (saccharose 25 g/l, mannite 8,5 g/l, meat extract 25 g/l, ammonia nitrate 0,4 g/l, pH 7,0) are inoculated with 5 % of the preculture. The fermentation is carried out at 32°C under the conditions described above. The paeciloquinones are detected in remarkable amounts after 4-5 days and are isolated from the supernatant.

b) 150 shake flasks with 4 baffles and 100 ml of the production medium CG-D 2.2 (saccharose 25 g/l, mannite 8,5 g/l, meat extract 10 g/l, peanut meal 20 g/l, ammonia nitrate 0,4 g/l, pH 7,0) are inoculated with 5 % of the preculture. The fermentation is carried out at 32°C under the conditions described above.The paeciloquinones can be detected in remarkable amounts after 4-5 days and isolated from the

supernatant.

Example 3: Fermentation in a bioreactor

For preparation of preculture 1 one slant (example 2) is used as inoculum for the first preculture with the components given in example 2. The preculture is incubated for 2 days at 28°C on a rotary shaker (250 rpm, amplitude 50 mm).

Preculture 2 is prepared by inoculation with 5 % of the preculture 1 and fermented under the same conditions. 2000 ml Erlenmeyer flasks with 1 baffle and 500 ml of the indicated medium are used.

a) 50 l bioreactor with blade stirrer; production medium CG-D 2.1 (example 2a) 30 l of the medium CG-D 2.1 are inoculated with 1,8 l of the preculture 2. The aeration rate is adjusted to 1 v/v/m, the impeller speed to 700 rpm. The fermentation temperature is 33°C. The $pO_2$ value which is adjusted to 100 % prior begin of fermentation decreases continuously to 82 % during the first 48 hours and increases to 97 % till the completion of fermentation after 120 hours. The pH raises from 7 to 7,4 after 24 hours, decreases to 6,7 after 72 hours and reaches about 8,1 after 120 hours. The various paeciloquinones reach their maximal titers after 72 to 120 hours and can be isolated from the supernatant.

b) 50 l bioreactor with blade stirrer; production medium CG-D 2.2 (example 2b)). 30 l of the medium CG-D 2.2 are inoculated with 1,8 l of the preculture 2. The aeration rate is adjusted to 1 v/v/m, the impeller speed to 700 rpm. The fermentation temperature is 33°C. Under these conditions the paeciloquinones reach their maximal titers after 96 to 120 hours. Paeciloquinone A and versiconol (Hatsuda, Y. et al., Agr. Biol. Chem. (Tokyo) 33 (1), 131-133 (Eng), 1969) are formed in significantly smaller amounts than under the conditions given in a).

Example 4: Isolation of Paeciloquinones A, B, C, D and versiconol.

The whole culture broth of 128 shake flasks (12.8 liters, example 2a)) is centrifuged. The pH of culture solution is adjusted to 5.0 and extracted with ethyl acetate (44 liters). The organic layer is washed with NaCl solution (20%) and concentrated in vacuo to give a red solid. The solid is dissolved in ethyl acetate - MeOH 3.5:1 and adsorbed on silica gel (LiChroprep Si60, 15-25 μm). After removal of the polar solvents *in vacuo* the solid material is applied on top of a column and separated into five fractions by column chromatography (LiChroprep Si60, 15-25 μm; 920 ml;

$CH_2Cl_2$ - MeOH (98:2, 700 ml), (95:5, 4 liters), (90:10, 2 liters), (85:15, 2 liters), (75:25, 1 liter), all solvents water saturated) which are combined according to UV trace (290nm) and HPLC (see below):

Fraction 1 (3.1 - 4.5 liters; a red solid) is crystallized twice from MeOH to give paeciloquinone A as yellow crystals with MP>350° (subl.).

Fraction 2 (4.5 - 5.7 liters), a part of which is rechromatographed on reversed phase (LiChroprep RP-18, 15-25 μm, conically shaped Büchi column;solvent A: water - TFA (100:0.1); solvent B:

acetonitrile - water - TFA (80:20:0.07); gradient from 40% B to 75% B in 35 minutes then to 50 % B in 30 min.; 30 ml/minute; sample dissolved in DMSO- MeOH 50:1 to give paeciloquinone C ($t_R$ 20-22.5 minutes) as a orange solid with MP>300° and paeciloquinone A ($t_R$ 27-32.4 minutes) as a yellow crystals after recrystallization from MeOH.

Fraction 3 (6.5 - 6.9 liters) is crystallized from a mixture of MeOH and ethyl acetate to give paeciloquinone B as orange crystals (MP 240-260°).

Fraction 4 (6.9 - 7.9 liters) contains paeciloquinone B and versiconol. Pure versiconol is obtained from similar material by silica gel HPLC chromatography ($CH_2Cl_2$ - EtOH (92:8) saturated with water; 8 ml/minutes; 290 nm; $t_R$ 12 minutes; sample injected in ethyl acetate ) to give versiconol after crystallization from MeOH (MP 245-60°).

Fraction 5 (8.3 - 9.5 liters) is crystallized from MeOH to give paeciloquinone D as orange crystals with MP>300°.

The NMR data of the isolated paeciloquinones are given in Tables 1 and 2.

Example 5: Isolation of Paeciloquinones B, E and F.

The whole culture broth of 150 shake flasks containing peanut meal (example 2b; 15 liters) is centrifuged. The pH of culture solution is adjusted to 5.0 and extracted with ethyl acetate (32 liters). The organic layer is washed with NaCl solution (20%) and concentrated *in vacuo* to give a red solid.

The solid is dissolved in ethyl acetate-MeOH 3:1 and adsorbed on silica gel (LiChroprep Si60, 15-25 μm). After removal of the polar solvents *in vacuo* the solid material is applied on top of a column and is

separated into two fractions by column chromatography (LiChroprep Si60, 15-25 µm; 920 ml; $CH_2Cl_2$ - MeOH (99:1, 500 ml), (98:2, 2 liters), (95:5, 2 liters), (90:10, 4 liters), (85:15, 2 liters), all solvents water saturated) which are combined according to UV trace (290nm) and HPLC (see below):
Fraction 1 (1.1 - 2.4 liters) is crystallized from MeOH to give paeciloquinone E as orange crystals (MP 212-214°).
Fraction 2 (4.7 - 10.5 liters) is rechromatographed on reversed phase (LiChroprep RP-18, 15-25 µm, conically shaped Büchi column;solvent A: water - TFA (100:0.1); solvent B: acetonitrile - water - TFA (80:20:0.07); gradient from 40 % B to 75 % B in 35 minutes then to 50 % B in 30 min.; 30 ml/minute; sample dissolved in MeOH- water 1:2 to give paeciloquinone B ($t_R$ 17.7-24 minutes) as a orange solid after removal of the acetonitrile *in vacuo* and crystallization from the remaining water and paeciloquinone F ($t_R$ 30.3-33.6 minutes) after extraction with $CH_2Cl_2$, removal of the organic solvent *in vacuo* as a yellow crystals (MP 201-205° from $CH_2Cl_2$/EtOH). The NMR-data of the isolated paeciloquinones are given in Tables 1 and 2.

Example 6: 1,3,6- and 3,6,8-Trimethoxy-paeciloquinone B methyl ester

To a stirred suspension of paeciloquinone B (30 mg) in methanol (3 ml) an ethereal solution of $CH_2N_2$ is added (10 ml; L. F. Fieser & M. Fieser, Reagents for Organic Synthesis, Vol. 1, Wiley, 1967, p 191, procedure b) The clear solution is stirred for 30 minutes and the solvent is removed by a stream of nitrogen. The pure products are obtained by semipreparative silica gel HPLC chromatography ($CH_2Cl_2$ - 2PrOH (99.5:0.5) saturated with water; 8 ml/minutes; 295nm; 4 runs) to give 1,3,6-trimethoxy-paeciloquinone B methyl ester ($t_R$ 3.7 minutes; MP 160-163°) and 3,6,8-trimethoxy-paeciloquinone B methyl ester ($t_R$ 5.3 minutes; MP>210°).

Table 1: $^{13}$C-NMR data of Paeciloquinones A,B,D,E and F. For each compound, numeration within the anthraquinone body C-atoms 1 to 8 (cf. column 1) refers to the numeration as indicated in formula III, which also gives the numeration within the $R_2$-substituent (cf. formula I) for paeciloquinones A,B and D. For paeciloquinone F, C-atom 1' corresponds to $C_5$ of the 2,3-epoxy-3-carboxy-pentan-2,5-diyl group. Numeration for paeciloquinone E is indicated in formula II above. Chemical shifts given in ppm. Solvent: DMSO-d6. Temperature: ambient except for [a] at 60°. Assignments with asterisks may be interchanged.

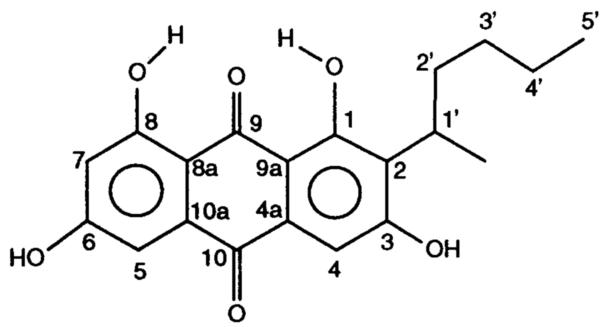

| Paecilochinon | A | B | D[a)] | E | F |
|---|---|---|---|---|---|
| 1 | 161.7 | 161.8 | 162.5* | 160.0 | 158.1* |
| 2 | 117.7 | 119.9 | 118.8 | 125.0 | 118.9 |
| 3 | 162.7 | ≈163 | ≈171 * | 161.9 | 157.9* |
| 4 | 108.0d | 108.0 d | 113.2 d | 110.1 | 108.1* |
| 4a | 133.1 | 132.6 | 132.5 | 132.6 | 133.8 |
| 5 | 108.7 d | 108.9 d | 108.0 d | 109.2 | 109.3 d |
| 6 | 165.1 | 165.3 | 163.8 | 165.7 | 165.3 |
| 7 | 107.8 d | 108.3 d | 108.0 d | 108.0 | 108.0 |
| 8 | 164.2 | 164.1 | 164.3 | 164.3 | 164.3 |
| 8a | ≈108.7 | 108.3 | 109.0 | 109.1 | 109.1* |
| 9 | 188.7 | 188.8 | 186.9 | 188.4 | 188.9 |
| 9a | 108.3 | 108.6 | ≈108.3 | 108.4 | 108.7* |
| 10 | 181.2 | 181.3 | 182.0 | 180.9 | 180.6 |
| 10a | 134.9 | 134.9 | 135.0 | 134.9 | 134.9 |
| 1'-CO | 176.7 | 173.7 | 175.6 | | |
| 1' | 34.6 d | 38.3 d | 40.9 t | 31.6 | 37.2 t |
| 2' | 27.1 t | 22.7 t | 33.9 d | 24.2 | 36.9 t |
| 3' | 66.7 t | 40.3 t | 59.6 d | 27.6 | 83.7 s |
| 4' | | 207.8 | | 105.1 | 108.9 |
| 5' | | 29.7 q | | 28.5 | 22.9 q |
| 1'-$CH_2$ | | | | 68.9 | |
| 3'-CO | | | | | 168.1 |

Table 2: $^1$H-NMR Data.
The allocation of the signals (column 1) refers to the numeration of C-atoms indicated in Table 1.

| Paeciloquinone | A | B | D$^{a)}$ | E$^{b)}$ | F |
|---|---|---|---|---|---|
| 1-OH | 12.80 s br* | 12.71 s* | | 12.81 b* | 12.68 b* |
| 3-OH/6-OH | 11.5 br | 11.3-4 br | | 12.08 b* | 12.0/11.4 b* |
| 4 | 7.20 s | 7.23 s | 6.90 s br | 6.98 s | 7.02 s |
| 5 | 7.11 d | 7.12 d | 7.08 d | 7.09 d | 7.12 d |
| 7 | 6.59 d | 6.59 d | 6.53 d | 6.56 d | 6.59 d |
| 8-OH | 12.18 s br* | 12.14 s* | 12.4 b | | |
| 1'-$CH_2$, -COOH | | | | 4.18/4.07 dd/dt | 13.36 b |
| 1' | 4.48 m | 3.95 dd | 4.07 dd | 3.55 m br. | 2.6/2.3 m |
| 2' | 2.43 m | 2.28/1.88 m | 2.15/1.90 m | 2.35-1.9 m | |
| 3' | 4.35 | 2.4/2.28 m | 3.34 m | | |
| 5' | | 2.00 s | | 1.49 s | 1.72 s |

Chemical shifts given in ppm. Solvent: DMSO-d6 except for $^{b)}$: DMSO-d6 - $C_6D_6$ mixture.
Temperature: ambient except for $^{a)}$ at 60°. Assignments with asterisks may be interchanged.

Example 7: Paeciloquinone B methyl ester

A solution of paeciloquinone B (76 mg) in methanol/$BF_3$ reagent (1.3M; 4 ml) is heated for 2 hours at 65°. The reaction is poured on ice, almost neutralized with a saturated $Na_2CO_3$ solution (pH 6) and extracted with ethyl acetate (50 ml). The organic phase is filtered to remove water and the solvent removed in vacuo. The almost pure product (90 %) is dissolved in 2-PrOH (100 µl), EtOAc (400 µl), $CH_2Cl_2$ (500 µl) and chromatographed on the semipreparative silica gel HPLC column ($CH_2Cl_2$ - EtOH (95:5) saturated with water; 8 ml/minutes; 290 nm; 10 runs; $t_R$ 14.3 minutes) to give Paeciloquinone B methyl ester (P>210°).

Example 8: 4'-Dihydro-paeciloquinone B

4'-Dihydropaeciloquinone is the compound of the formula I wherein $R_1$, $R_3$, $R_4$ and $R_5$ are each hydrogen and $R_2$ is $CH(R_6)-(CH_2)_2-X-CH_3$ wherein $R_6$ is carboxy and X is CHOH. To a stirred solution of paeciloquinone B (20 mg) in dry THF (3 ml) a solution of $LiAlH_4$ in THF (250 µl, 1M; Aldrich) is added with a syringe. The solution which turns deep red is stirred for 30 minutes at 50°. The reaction is stopped by addition of ethyl acetate (200 µl) and ice. The mixture is acidified with 2N HCl and distributed between water and $CH_2Cl_2$. The organic phase is filtered to remove water and the solvent removed in vacuo. The title compound (MP 164-167°) is obtained by semipreparative silica gel HPLC chromatography ($CH_2Cl_2$ - EtOH (85:15) saturated with water; 8 ml/minutes; 285nm; 6 runs; $t_R$ 19-25 minutes).

Example 9: 1,3,6- and 3,6,8-Trimethoxy-paeciloquinone D methyl ester

To a stirred suspension of paeciloquinone D (8.7 mg) in methanol (1 ml) an ethereal solution of $CH_2N_2$ is added (5 ml; L.F. Fieser & M. Fieser, Reagents for Organic Synthesis, Vol. 1, Wiley, 1967, p 191, procedure b). The clear solution is stirred for 30 minutes and the solvent is removed by a stream of nitrogen. The pure products are obtained by semipreparative silica gel HPLC chromatography ($CH_2Cl_2$ - 2PrOH (98.8:1.2) saturated with water; 8 ml/minutes; 285nm; 2 runs) to give 1,3,6-Trimethoxy-paeciloquinone D methyl ester ($t_R$ 5.8 minutes; MP 124-130°) and 3,6,8-Trimethoxy-paeciloquinone D methyl ester ($t_R$ 8.0 minutes; MP 173-176°).

Example 10: 1,3,6-Trimethoxy-paeciloquinone A

To a stirred suspension of paeciloquinone A (25 mg) in methanol (2 ml) an ethereal solution of $CH_2N_2$ is added (5 ml; L.F. Fieser & M. Fieser, Reagents for Organic Synthesis, Vol. 1, Wiley, 1967, p 191, procedure b). The clear solution is stirred for 7 minutes and the solvent is removed by a stream of nitrogen. The pure product (P>210°) is obtained by semipreparative silica gel HPLC chromatography ($CH_2Cl_2$, saturated with water; 8 ml/minutes; 300 nm; 5 runs; $t_R$ 4.7 minutes).

Example 11: 1,6-Dimethoxy-paeciloquinone F methyl ester and 1,6,8-Trimethoxy-paeciloquinone F methyl ester

To a stirred suspension of paeciloquinone F (20 mg) in methanol (2 ml) an ethereal solution of $CH_2N_2$ is added (10 ml; L.F. Fieser & M. Fieser, Reagents for Organic Synthesis, Vol. 1, Wiley, 1967, p 191, procedure b). The clear red solution is stirred for 15 min. and the solvent is removed by a stream of nitrogen. The pure products are obtained by semipreparative silica gel HPLC chromatography ($CH_2Cl_2$ - heptane (80:20), saturated with water; 8 ml/minutes; 285 nm; 8 runs) to give 1,6-dimethoxy- paeciloquinone F methyl ester ($t_R$ 3.6 minutes) and 1,6,8-trimethoxy-paeciloquinone F methyl ester ($t_R$ 6.6 minutes; MP>210°).

Example 12: Pharmaceutical Preparation

5000 capsules are prepared, each comprising 0.25 g of active ingredient, for example anyone of the compounds prepared in Examples 1 to 11:

| Composition | |
|---|---|
| active ingredient | 1250 g |
| talc | 180g |
| wheat starch | 120 g |
| magnesium stearate | 80 g |
| lactose | 20g |

Method: The pulverulent substances are forced through a sieve having a mesh size of 0.6 mm and mixed together. 0.33 g portions of the mixture are filled into gelatin capsules using a capsule-filling machine.

Deposition data

Paecilomyces carneus P-177 was deposited with the Deutsche Sammlung für Mikroorganismen und Zellkulturen (DSM), Marscheroder Weg 1B, D-3300 Braunschweig under accession no. DSM 7243 on September 16, 1992.

**Claims**

**1.** A compound of the formula

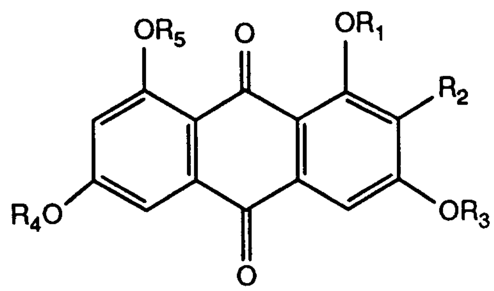

(I),

wherein $R_1$ is hydrogen or alkyl;
$R_2$ is 2-oxo-tetrahydrofur-3-yl, $CH(R_6)-(CH_2)_2-X-CH_3$ wherein $R_6$ is carboxy or alkoxycarbonyl and X is C(=O) or $CH(OR_7)$ wherein $R_7$ is hydrogen or alkyl, or $R_2$ is $CH(R_6)-CH_2-CH_2-OR_7$ wherein $R_6$ and $R_7$ have the meanings given above;
and $R_3$, $R_4$ and $R_5$ are each independently of the other hydrogen or alkyl;
or wherein $R_1$, $R_4$ and $R_5$ have the meanings given above and $R_2$ and $R_3$ together form 2,3 epoxy-3-carboxy-pentan-2,5-diyl or 2,3 epoxy-3-alkoxycarbonyl-pentan-2,5-diyl, the $C_2$-atom of said pentan- 2,5-diyl binding to the oxygen;
or wherein the partial structure

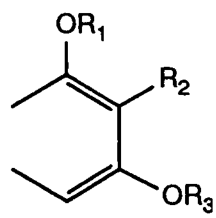

represents the group of the formula

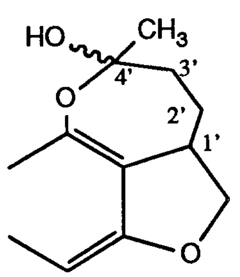

(II);

or a salt thereof.

**2.** A compound of the formula I according to claim 1 wherein $R_1$ is hydrogen or alkyl; $R_2$ is 2-oxo-tetrahydrofur-3-yl, $CH(R_6)$-$(CH_2)_2$-X-$CH_3$ wherein $R_6$ is carboxy or lower alkoxycarbonyl and X is C( = O) or $CH(OR_7)$ wherein $R_7$ is hydrogen or alkyl or $R_2$ is $CH(R_6)$-$CH_2$-$CH_2$-$OR_7$ wherein $R_6$ and $R_7$ have the meanings given above; and $R_3$, $R_4$ and $R_5$ are each independently of the other hydrogen or alkyl; or wherein $R_1$, $R_4$ and $R_5$ have the meanings given above and $R_2$ and $R_3$ together form 2,3-epoxy-3-carboxy-pentan-2,5-diyl or 2,3 epoxy-3-alkoxycarbonyl-pentan-2,5-diyl; with the provisio that at least either of $R_1$ or $R_5$ is hydrogen, or a salt thereof.

**3.** A compound of the formula I according to claim 1, wherein $R_1$ is hydrogen or methyl; $R_2$ is 2-oxo-tetrahydrofur-3-yl, $CH(R_6)$-$(CH_2)_2$-X-$CH_3$ wherein $R_6$ is carboxy or methoxycarbonyl and X is C( = O) or $CH(OR_7)$ wherein $R_7$ is hydrogen or methyl, or $R_2$ is $CH(R_6)$-$CH_2$-$CH_2$-$OR_7$ wherein $R_6$ and $R_7$ have the meanings given above; and $R_3$, $R_4$ and $R_5$ are each independently of the other hydrogen or methyl; or wherein $R_1$, $R_4$ and $R_5$ have the meanings given above and $R_2$ and $R_3$ together form 2,3 epoxy-3-carboxy-pentan-2,5-diyl or 2,3 epoxy-3-alkoxycarbonyl-pentan-2,5-diyl; with the provisio that at least either of $R_1$ or $R_5$ is hydrogen; or a salt thereof.

**4.** A compound of the formula I according to claim 1, wherein $R_1$ is hydrogen ; $R_2$ is 2-oxo-tetrahydrofur-3-yl, $CH(R_6)$-$(CH_2)_2$-X-$CH_3$ wherein $R_6$ is carboxy and X is C( = O), or $CH(R_6)$-$CH_2$-$CH_2$-$OR_7$ wherein $R_6$ and $R_7$ have the meanings given above; and $R_3$, $R_4$ and $R_5$ are each hydrogen; or a salt thereof.

**5.** A compound of the formula I according to claim 1, wherein $R_1$, $R_3$, $R_4$ and $R_5$ are hydrogen and $R_2$ is $CH(R_6)$-$(CH_2)_2$-X-$CH_3$ wherein $R_6$ is carboxy X is C( = O); or a salt thereof.

**6.** A process for the production of a compound according to claim 1, said process comprising culturing strain Paecilomyces carneus P-177, or a culture derivable therefrom, in a suitable nutrient medium, isolating said compound from the culture broth and, optionally, converting said compound into another compound or into a salt thereof.

**7.** The strain Paecilomyces carneus P-177 Brown & Smith deposited with the Deutsche Sammlung für Mikroorganismen und Zellkulturen (DSM) in Braunschweig under accession no. DSM 7243 on September 16, 1992, or a strain derived therefrom.

**8.** A pharmaceutical composition comprising a compound of the formula I according to claim 1 together with a suitable pharmaceutical carrier.

**9.** A compound of the formula I or a pharmaceutically acceptable salt thereof according to claim 1 for use in a method for the treatment of the human or animal body.

**10.** Use of a compound of formula I according to claim 1 or of a pharmaceutically acceptable salt thereof for the preparation of a pharmaceutical composition.

**11.** A method of treating the human or animal body comprising the administration of a therapeutically effective amount of a compound of formula I as claimed in claim 1 or a pharmaceutically acceptable salt thereof.

European Patent Office

# EUROPEAN SEARCH REPORT

Application Number
EP 94 81 0039

## DOCUMENTS CONSIDERED TO BE RELEVANT

| Category | Citation of document with indication, where appropriate, of relevant passages | Relevant to claim | CLASSIFICATION OF THE APPLICATION (Int.Cl.6) |
|---|---|---|---|
| A | JOURNAL OF THE CHEMICAL SOCIRETY, PERKIN TRANSACTIONS 1<br>no. 2 , 1979 , LETCHWORTH GB<br>pages 451 - 459<br>P. S. STEVN ET AL 'Structure and carbon-13 nuclear megnetic resonance assignements of versiconal acetate, versiconol acetate and versiconol metabolites from cultures of Aspergillus parasiticus treated with dichlorvos'<br>* page 451 - page 452 *<br>----- | 1 | C07D493/06<br>C07C65/40<br>C07C69/738<br>C07D307/32<br>A61K31/19<br>A61K31/335<br>A61K31/215<br>C12P15/00<br>C12P17/04<br>C12P17/18<br>//(C07D493/06, 313:00, 307:00), (C12P17/18, C12R1:79) |

TECHNICAL FIELDS SEARCHED (Int.Cl.6)

C07D
C07C
A61K
C12P

The present search report has been drawn up for all claims

| Place of search | Date of completion of the search | Examiner |
|---|---|---|
| THE HAGUE | 24 June 1994 | Voyiazoglou, D |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

European Patent Office

## CLAIMS INCURRING FEES

The present European patent application comprised at the time of filing more than ten claims.

- [ ] All claims fees have been paid within the prescribed time limit. The present European search report has been drawn up for all claims.
- [ ] Only part of the claims fees have been paid within the prescribed time limit. The present European search report has been drawn up for the first ten claims and for those claims for which claims fees have been paid,

  namely claims:
- [x] No claims fees have been paid within the prescribed time limit. The present European search report has been drawn up for the first ten claims.

## LACK OF UNITY OF INVENTION

The Search Division considers that the present European patent application does not comply with the requirement of unity of invention and relates to several inventions or groups of inventions,

namely:

- [ ] All further search fees have been paid within the fixed time limit. The present European search report has been drawn up for all claims.
- [ ] Only part of the further search fees have been paid within the fixed time limit. The present European search report has been drawn up for those parts of the European patent application which relate to the inventions in respect of which search fees have been paid,

  namely claims:
- [ ] None of the further search fees has been paid within the fixed time limit. The present European search report has been drawn up for those parts of the European patent application which relate to the invention first mentioned in the claims,

  namely claims: